# EUROPEAN PATENT APPLICATION

(11) **EP 3 173 789 A1**
(43) Date of publication of application: **31.05.2017**
(21) Application number: 15195994.7
(22) Date of filing: 24.11.2015
(51) Int. Cl.: G01N 33/569, G01N 33/68, C40B 40/02, C40B 40/10, G01N 33/543

(54) **METHOD FOR DIFFERENTIATION OF IMMUNE RESPONSE IN AN INDIVIDUAL**

(71) Applicant: Helmholtz-Zentrum für Infektionsforschung GmbH, 38124 Braunschweig (DE)
(72) Inventor: Bohm, Katrin, 38448 Wolfsburg (DE); Sievers, Claudia, 49685 Emstek (DE); Krause, Gérard, 38124 Braunschweig (DE)
(74) Representative: Kröncke, Rolf

(57) **Abstract**

In a first aspect, the present invention relates to a method allowing differentiation between infection-based immune response against an infective agent and a vaccine induced immune response against an infective agent in an individual. Furthermore, a kit or a system is provided allowing differentiation between infection-based immune response against an infective agent and a vaccine induced immune response against an infective agent in an individual based on differentiation on antibodies present in the individual directed against linear epitopes and conformational epitopes. Finally, an array is provided, in particular, a microarray, for multiplex analysis containing different types of antigens.

SEQUENCE LISTING

<110>  Helmholtz Zentrum für Infektionsforschung GmbH

<120>  Method for the differentiation of immune response in an
       individual

<130>  3275-038 EP-1

<160>  6

<170>  PatentIn version 3.5

<210>  1
<211>  300
<212>  PRT
<213>  Hepatitis A virus

<400>  1

Val Gly Asp Asp Ser Gly Gly Phe Ser Thr Thr Val Ser Thr Glu Gln
1               5                   10                  15

Asn Val Pro Asp Pro Gln Val Gly Ile Thr Thr Met Lys Asp Leu Lys
            20                  25                  30

Gly Lys Ala Asn Arg Gly Lys Met Asp Val Ser Gly Val Gln Ala Pro
        35                  40                  45

Val Gly Ala Ile Thr Thr Ile Glu Asp Pro Val Leu Ala Lys Lys Val
    50                  55                  60

Pro Glu Thr Phe Pro Glu Leu Lys Pro Gly Glu Ser Arg His Thr Ser
65                  70                  75                  80

Asp His Met Ser Ile Tyr Lys Phe Met Gly Arg Ser His Phe Leu Cys
                85                  90                  95

Thr Phe Thr Phe Asn Ser Asn Asn Lys Glu Tyr Thr Phe Pro Ile Thr
            100                 105                 110

Leu Ser Ser Thr Ser Asn Pro Pro His Gly Leu Pro Ser Thr Leu Arg
        115                 120                 125

Trp Phe Phe Asn Leu Phe Gln Leu Tyr Arg Gly Pro Leu Asp Leu Thr
    130                 135                 140

Ile Ile Ile Thr Gly Ala Thr Asp Val Asp Gly Met Ala Trp Phe Thr
145                 150                 155                 160

Pro Val Gly Leu Ala Val Asp Thr Pro Trp Val Glu Lys Glu Ser Ala
                165                 170                 175

Leu Ser Ile Asp Tyr Lys Thr Ala Leu Gly Ala Val Arg Phe Asn Thr
        180                 185                 190

Arg Arg Thr Gly Asn Ile Gln Ile Arg Leu Pro Trp Tyr Ser Tyr Leu
        195                 200                 205

Tyr Ala Val Ser Gly Ala Leu Asp Gly Leu Gly Asp Lys Thr Asp Ser
    210                 215                 220

Thr Phe Gly Leu Val Ser Ile Gln Ile Ala Asn Tyr Asn His Ser Asp
225                 230                 235                 240

Glu Tyr Leu Ser Phe Ser Cys Tyr Leu Ser Val Thr Glu Gln Ser Glu
                245                 250                 255

Phe Tyr Phe Pro Arg Ala Pro Leu Asn Ser Asn Ala Met Leu Ser Thr
            260                 265                 270

Glu Ser Met Met Ser Arg Ile Ala Ala Gly Asp Leu Glu Ser Ser Val
        275                 280                 285

Asp Asp Pro Arg Ser Glu Glu Asp Lys Arg Phe Glu
    290                 295                 300

<210>  2
<211>  222
<212>  PRT
<213>  Hepatitis A virus

<400>  2

Asp Ile Glu Glu Glu Gln Met Ile Gln Ser Val Asp Arg Thr Ala Val
1               5                   10                  15

Thr Gly Ala Ser Tyr Phe Thr Ser Val Asp Gln Ser Ser Val His Thr
            20                  25                  30

Ala Glu Val Gly Ser His Gln Val Glu Pro Leu Arg Thr Ser Val Asp
        35                  40                  45

Lys Pro Gly Ser Lys Lys Thr Gln Gly Glu Lys Phe Phe Leu Ile His
    50                  55                  60

Ser Ala Asp Trp Leu Thr Thr His Ala Leu Phe His Glu Val Ala Lys
65                  70                  75                  80

Leu Asp Val Val Lys Leu Leu Tyr Asn Glu Gln Phe Ala Val Gln Gly
                85                  90                  95

Leu Leu Arg Tyr His Thr Tyr Ala Arg Phe Gly Ile Glu Ile Gln Val
            100                 105                 110

Gln Ile Asn Pro Thr Pro Phe Gln Gln Gly Gly Leu Ile Cys Ala Met
        115                 120                 125

Val Pro Gly Asp Gln Ser Tyr Gly Ser Ile Ala Ser Leu Thr Val Tyr
    130                 135                 140

Pro His Gly Leu Leu Asn Cys Asn Ile Asn Asn Val Val Arg Ile Lys
145                 150                 155                 160

Val Pro Phe Ile Tyr Thr Arg Gly Ala Tyr His Phe Lys Asp Pro Gln
                165                 170                 175

Tyr Pro Val Trp Glu Leu Thr Ile Arg Val Trp Ser Glu Leu Asn Ile
            180                 185                 190

Gly Thr Gly Thr Ser Ala Tyr Thr Ser Leu Asn Val Leu Ala Arg Phe
        195                 200                 205

Thr Asp Leu Glu Leu His Gly Leu Thr Pro Leu Ser Thr Gln
    210                 215                 220

<210>  3
<211>  246
<212>  PRT
<213>  Hepatitits A virus

<400>  3

Met Met Arg Asn Glu Phe Arg Val Ser Thr Thr Glu Asn Val Val Asn
1               5                   10                  15

Leu Ser Asn Tyr Glu Asp Ala Arg Ala Lys Met Ser Phe Ala Leu Asp
            20                  25                  30

Gln Glu Asp Trp Lys Ser Asp Pro Ser Gln Gly Gly Gly Ile Lys Ile
        35                  40                  45

Thr His Phe Thr Thr Trp Thr Ser Ile Pro Thr Leu Ala Ala Gln Phe
    50                  55                  60

Pro Phe Asn Ala Ser Asp Ser Val Gly Gln Gln Ile Lys Val Ile Pro
65                  70                  75                  80

Val Asp Pro Tyr Phe Phe Gln Met Thr Asn Thr Asn Pro Asp Gln Lys
                85                  90                  95

Cys Ile Thr Ala Leu Ala Ser Ile Cys Gln Met Phe Cys Phe Trp Arg
            100                 105                 110

Gly Asp Leu Val Phe Asp Phe Gln Val Phe Pro Thr Lys Tyr His Ser
        115                 120                 125

Gly Arg Leu Leu Phe Cys Phe Val Pro Gly Asn Glu Leu Ile Asp Val
    130                 135                 140

Ser Gly Ile Thr Leu Lys Gln Ala Thr Thr Ala Pro Cys Ala Val Met
145                 150                 155                 160

Asp Ile Thr Gly Val Gln Ser Thr Leu Arg Phe Arg Val Pro Trp Ile
                165                 170                 175

Ser Asp Thr Pro Tyr Arg Val Asn Arg Tyr Thr Lys Ser Ala His Gln
            180                 185                 190

Lys Gly Glu Tyr Thr Ala Ile Gly Lys Leu Ile Val Tyr Cys Tyr Asn
        195                 200                 205

Arg Leu Thr Ser Pro Ser Asn Val Ala Ser His Val Arg Val Asn Val
    210                 215                 220

Tyr Leu Ser Ala Ile Asn Leu Glu Cys Phe Ala Pro Leu Tyr His Ala
225                 230                 235                 240

Met Asp Val Thr Thr Gln
                245

<210> 4
<211> 189
<212> PRT
<213> Hepatitis A virus

<400> 4

Ser His Ile Glu Cys Arg Lys Pro Tyr Lys Glu Leu Arg Leu Glu Val
1               5                   10                  15

Gly Lys Gln Arg Leu Lys Tyr Ala Gln Glu Glu Leu Ser Asn Glu Val
            20                  25                  30

Leu Pro Pro Pro Arg Lys Met Lys Gly Leu Phe Ser Gln Ala Lys Ile
        35                  40                  45

Ser Leu Phe Tyr Thr Glu Glu His Glu Ile Met Lys Phe Ser Trp Arg
    50                  55                  60

Gly Val Thr Ala Asp Thr Arg Ala Leu Arg Arg Phe Gly Phe Ser Leu
65                  70                  75                  80

Ala Ala Gly Arg Ser Val Trp Thr Leu Glu Met Asp Ala Gly Val Leu
                85                  90                  95

Thr Gly Arg Leu Ile Arg Leu Asn Asp Glu Lys Trp Thr Glu Met Lys
            100                 105                 110

Asp Asp Lys Ile Val Ser Leu Ile Glu Lys Phe Thr Ser Asn Lys Tyr
        115                 120                 125

Trp Ser Lys Val Asn Phe Pro His Gly Met Leu Asp Leu Glu Glu Ile
    130                 135                 140

Ala Ala Asn Ser Lys Asp Phe Pro Asn Met Ser Glu Thr Asp Leu Cys
145                 150                 155                 160

Phe Leu Leu His Trp Leu Asn Pro Lys Lys Ile Asn Leu Ala Asp Arg
                165                 170                 175

Met Leu Gly Leu Ser Gly Val Gln Glu Ile Lys Glu Gln
            180                 185

<210>  5
<211>  335
<212>  PRT
<213>  Hepatitis A virus

<400>  5

Ser Phe Ser Asn Trp Leu Arg Asp Ile Cys Ser Gly Ile Thr Ile Phe
1               5                   10                  15

Lys Asn Phe Lys Asp Ala Ile Tyr Trp Leu Tyr Thr Lys Leu Lys Asp
            20                  25                  30

Phe Tyr Glu Val Asn Tyr Gly Lys Lys Lys Asp Ile Leu Asn Ile Leu
        35                  40                  45

Lys Asp Asn Gln Gln Lys Ile Glu Lys Ala Ile Glu Glu Ala Asp Glu
    50                  55                  60

Phe Cys Ile Leu Gln Ile Gln Asp Val Glu Lys Phe Glu Gln Tyr Gln
65                  70                  75                  80

Lys Gly Val Asp Leu Ile Gln Lys Leu Arg Thr Val His Ser Met Ala
                85                  90                  95

Gln Val Asp Pro Asn Leu Met Val His Leu Ser Pro Leu Arg Asp Cys
            100                 105                 110

Ile Ala Arg Val His Gln Lys Leu Lys Asn Leu Gly Ser Ile Asn Gln
        115                 120                 125

Ala Met Val Thr Arg Cys Glu Pro Val Val Cys Tyr Leu Tyr Gly Lys
    130                 135                 140

Arg Gly Gly Gly Lys Ser Leu Thr Ser Ile Ala Leu Ala Thr Lys Ile
145                 150                 155                 160

Cys Lys His Tyr Gly Val Glu Pro Glu Lys Asn Ile Tyr Thr Lys Pro
                165                 170                 175

Val Ala Ser Asp Tyr Trp Asp Gly Tyr Ser Gly Gln Leu Val Cys Ile
            180                 185                 190

Ile Asp Asp Ile Gly Gln Asn Thr Thr Asp Glu Asp Trp Ser Asp Phe
        195                 200                 205

Cys Gln Leu Val Ser Gly Cys Pro Met Arg Leu Asn Met Ala Ser Leu
    210                 215                 220

Glu Glu Lys Gly Arg His Phe Ser Ser Pro Phe Ile Ile Ala Thr Ser
225                 230                 235                 240

Asn Trp Ser Asn Pro Ser Pro Lys Thr Val Tyr Val Lys Glu Ala Ile
                245                 250                 255

Asp Arg Arg Leu His Phe Lys Val Glu Val Lys Pro Ala Ser Phe Phe
            260                 265                 270

Lys Asn Pro His Asn Asp Met Leu Asn Val Asn Leu Ala Lys Thr Asn
        275                 280                 285

Asp Ala Ile Lys Asp Met Ser Cys Val Asp Leu Ile Met Asp Gly His
    290                 295                 300

Asn Val Ser Leu Met Asp Leu Leu Ser Ser Leu Val Met Thr Val Glu
305                 310                 315                 320

Ile Arg Lys Gln Asn Met Thr Glu Phe Met Glu Leu Trp Ser Gln
                325                 330                 335

<210>  6
<211>  219

<212>  PRT
<213>  Hepatitis A virus

<400>  6

Ser Thr Leu Glu Ile Ala Gly Leu Val Arg Lys Asn Leu Val Gln Phe
1               5                   10                  15

Gly Val Gly Glu Lys Asn Gly Cys Val Arg Trp Val Met Asn Ala Leu
            20                  25                  30

Gly Val Lys Asp Asp Trp Leu Leu Val Pro Ser His Ala Tyr Lys Phe
        35                  40                  45

Glu Lys Asp Tyr Glu Met Met Glu Phe Tyr Phe Asn Arg Gly Gly Thr
    50                  55                  60

Tyr Tyr Ser Ile Ser Ala Gly Asn Val Val Ile Gln Ser Leu Asp Val
65                  70                  75                  80

Gly Phe Gln Asp Val Val Leu Met Lys Val Pro Thr Ile Pro Lys Phe
                85                  90                  95

Arg Asp Ile Thr Gln His Phe Ile Lys Lys Gly Asp Val Pro Arg Ala
            100                 105                 110

Leu Asn Arg Leu Ala Thr Leu Val Thr Thr Val Asn Gly Thr Pro Met
        115                 120                 125

Leu Ile Ser Glu Gly Pro Leu Lys Met Glu Glu Lys Ala Thr Tyr Val
    130                 135                 140

His Lys Lys Asn Asp Gly Thr Thr Val Asp Leu Thr Val Asp Gln Ala
145                 150                 155                 160

Trp Arg Gly Lys Gly Glu Gly Leu Pro Gly Met Cys Gly Gly Ala Leu
                165                 170                 175

Val Ser Ser Asn Gln Ser Ile Gln Asn Ala Ile Leu Gly Ile His Val
            180                 185                 190

Ala Gly Gly Asn Ser Ile Leu Val Ala Lys Leu Val Thr Gln Glu Met
        195                 200                 205

Phe Gln Asn Ile Asp Lys Lys Ile Glu Ser Gln
    210                 215

## Description

In a first aspect, the present invention relates to a method allowing differentiation between infection-based immune response against an infective agent and a vaccine induced immune response against an infective agent in an individual. Furthermore, a kit or a system is provided allowing differentiation between infection-based immune response against an infective agent and a vaccine induced immune response against an infective agent in an individual based on differentiation on antibodies present in the individual directed against linear epitopes and conformational epitopes. Finally, an array is provided, in particular, a microarray, for multiplex analysis containing different types of antigens.

### Prior Art

Human pathogens remain a major public health concern in a society. Specific treatment for viruses is only available for few viruses and increasing spread of antimicrobial and antiviral resistance has developed to a global threat. Undoubtedly the most effective intervention to address this challenge is vaccination. Clinical studies can use conventional immunological biomarkers to measure the effectivity of a new or an established vaccine. Measuring community effectiveness of a vaccination campaign, however, encounters major methodological challenges: 1) clinical features of most infections are not pathognomonic enough to allow syndromic measurements as indicator for one unique pathogen, 2) existing immunological markers of vaccine preventable pathogens do not allow the distinction between immunological response to natural infection on one hand and response to vaccination on the other hand. This still remains true for all vaccine preventable pathogens except for hepatitis B, where detection of anti-HBs in absence of HBs-Ag and anti-HBC clearly indicates immunity due to vaccination. Such indicative pattern is not applicable to other vaccine preventable pathogens as of yet.

A serological test, able to distinguish between immune response to natural infection and that to vaccination would be of immense benefit for assessing the effectiveness of vaccination strategies. The need for such a tool is particularly urgent in the context of a) epidemics of pathogens with declining sporadic incidence such as Hepatitis A in Europe, b) large vaccination campaigns in a phase of epidemiological transition such as is the case for Hepatitis A in Latin America and c) anticipated introduction of newly developed vaccines such as Hepatitis E in China, d) vaccines of fairly short termed effectivity due to seasonal variations of viral strains with differing antigenic characteristics, as typically know for influenza. By far the most obvious and most ground breaking impact of such a serological tool is to be expected for e) pathogens which are target of global elimination goals, such as measles.

Even the few aforementioned viruses represent a broad scope of species and vaccine designs:
Hepatitis A Virus is a picornavirus, leading to an increasing number of outbreaks in the European Union in recent years and causing a large proportion of hepatic failure among children in various Latin American countries (Tanaka J. (2000) Vaccine, Volume 18, Suppl 1, 2000, S57-S60). The vaccine currently used is based on an inactivated hepatitis A virus.

Hepatitis E is a yet unclassified RNA virus. WHO estimates 20 million hepatitis E infections with over 3 million symptomatic cases, and 56 600 hepatitis E-related deaths globally. In China a vaccine against HEV has been developed and licensed, which is based on recombinant virus like particles, but still is not included in national vaccination strategies. (. Li, J. Zhang, et al., (2005) Vaccine, 23, pp. 2893-2901).

Influenza viruses are known to cause enormous burden of diseases. The effectiveness of influenza vaccines, currently based on surface antigens (hemaggluttinin and neuraminidase) of inactivated viruses, are challenged by the seasonal variation or strains and the variability of their respective surface antigens. Measles, the most prominent and urgent WHO-target for eradication is a paramyxovirus, yearly causing over 100.000 deaths globally. The vaccine is a live attenuated vaccine based on the Edmonston-Enders strain. Mumps is also a paramyxovirus, whose life attenuated form is included in the MMR vaccine, estimated to globally cause over 140.000 deaths every year. Rubella belongs to the togavirus, whose life attenuated form is the third component of the MMR vaccine. The congenital rubella syndrome results in congenital defects or death. WHO estimates that despite widespread vaccination coverage still 100.000 babies are born with CRS every year worldwide.

Existing assays for serological investigations of active infections whereby antibodies are determined can only measure the presence of one type of antibody against said infective agent. That is, the presently available assays allow measuring the presence of one type of antibody only. In addition, the presently available assays do not allow differentiating between infection based immune response against the infective agent and vaccine induced immune response in said individual. However, for determining the level of endemic infection and the success of endemic vaccination, a tool is desired for differentiating natural immune response and vaccine induced immune response in individuals.

That is, for epidemiological studies, e.g. outbreak of infection and level of endemic infection as well as determining the level of vaccination success, screening assays are desired allowing differentiation between these two types of immune response in an individual, in particular, allowing high throughput screening of these individuals at low costs and requiring small amounts of serum.

That is, not only for individual diagnosis of infection, but also in population based serosurveys, these tools and methods may be useful. These methods and tools would be particularly valuable for measuring the impact of recently initiated vaccine campaigns in different parts of the world, e.g. Latin America.

Stewart, D.R.S. et al, 1997, The Journal of Infectious Diseases, 176, 593-601 describe the detection of antibodies to the nonstructural 3C proteinase of hepatitis A virus. Therein, it is speculated that use of the 3C proteinase as an antigen can accurately detect antibodies to a non-structural protein and therefore distinguish an immune response to active infection from that resulting from vaccination with an inactivated vaccine since allegedly, the vaccination with inactivated virus should not raise antibodies against the 3C proteinase. However, the present inventors failed to rework the same. Of note, the experiments are based on experimental infection in chimpanzees and not natural infection. Further, Robertson B.H. et al, Journal of Medical Virology, 1993, 40:76-82 describe antibody response to non-structural proteins of hepatitis A virus following infection. It is discussed therein that the nonstructural proteins of HAV of the P2 region of the viral DNA may be used to differentiate between natural infection and vaccination. It is discussed that the nonstructural proteins may serve as markers for active replication and infection with HAV. Moreover, it is noted that animals immunized with inactivated or life attenuated HAV had no evidence of nonstructural antibodies after immunization or challenge with wild type virus. The authors conclude that nonstructural proteins may allow differentiation between inactivated vaccine induced antibodies and natural infection. However, the system is based on *in vitro* translated structures and the specificity and sensitivity is 87 % and 84 %, respectively only.

Waterboer T. et al, Clin. Chem. 2005, 51:10, 1845-1853 describe multiplex human papillomavirus serology based on in situ-purified glutathione s-transferase fusion proteins. Therein, a multiplex serology is described enabling antibody analysis of large numbers of sera against up to 100 antigens in parallel having a potential to replace ELISA technology. The technology applied is based on the Luminex technology, namely, the xMAP technology.

In Clavijo, A., et al., (2006), Vaccine 24, 1693-1704, simultaneous detection of antibodies to foot-and-mouth disease using non-structural proteins 3ABC, 3D, 3A and 3B by a multiplexed Luminex assay to differentiate infected from vaccinated cattle is described. Therein differentiation should be possible due to the presence or absence of autoantibodies against structural or non-structural proteins. That is, thus far, the approaches are based on the differentiation between structural and non-structural proteins based on the theory that natural infection results in antibodies directed against both, structural and non-structural proteins while vaccine based immune response results in an antibody response against structural proteins only. However, validation of the same failed.

Hence, there is still a need to provide a method and a serological tool for the detection of antibodies allowing differentiation between natural and vaccine based immune response in an individual, thus, allowing identification of the immune response inducing agent.

### Brief description of the present invention

In a first aspect, the present invention relates to a method for differentiation between infection-based immune response against an infective agent and a vaccine induced immune response against an infective agent in an individual whereby the vaccine is a vaccine based on the infective agent containing an antigen having conformational epitope(s), comprising the steps of:
- providing multiple of antigens derived from the infective agent wherein at least one antigen is provided having a linear epitope and at least one antigen is provided having a conformational epitope whereby the conformational epitope is present in the vaccine;
- allowing formation of binding pairs of an antibody present in a sample obtained from the individual with one of the multiple of antigens provided;
- determining binding pairs of antigen/antibody formed in said sample by an immunological method;
- differentiating infection based immune response and vaccine based immune response against the infective agent i) based on the presence and/or amount of binding pairs of antigen/antibody whereby the antigen has a linear epitope, and ii) based on the presence and/or amount of binding pairs of antigen/antibody whereby the antigen has the conformational epitope;
wherein the determination of the binding pairs is with the same type of immunological method, preferably within a single assay.

In particular, the method differentiates between an immunization with attenuated or inactivated virus and natural infection of said virus.

In a further aspect, the present invention relates to a kit or a system for differentiating between infection based immune response against an infective agent and a vaccine induced immune response against an infective agent in an individual, comprising multiple of antigens derived from the infective agent wherein at least one antigen is provided having a linear epitope and at least one antigen is provided having a conformational epitope whereby the conformational epitope is present in the vaccine; means for determining antibodies present in a sample of an individual whereby the antibodies are determined as binding pairs of antigens and antibodies and whereby said means have optionally a marker moiety for measuring the presence and/or amount of the formed binding pair of antigen/antibody; and, optionally, means for binding the antigens.

Finally, the present invention provides an array, like a microarray, for multiplex analysis, containing at least two antigens derived from an infective agent wherein at least one antigen is an antigen having a linear epitope and wherein at least one antigen is an antigen having a conformational epitope of said infective agent, and said array, like said microarray, is a microsphere based array, like a microsphere based microarray.

### Brief description of the figures

Figure 1: The flow chart shows the validation process with which the specific antigen cutoffs as well as the assay cutoff are calculated.
Figure 2: Boxplot for sera with known status in HAV serology.

Depicted is the distribution of the mean fluorescence intensity (MFI) for 57 HAV negative sera (0), 32 HAV infected sera (1) and 42 HAV vaccinated sera (2) for antibodies against the inactivated virus (HAV) and the 6 recombinant antigens (VP1 - 3C). The figure shows that only inactivated virus allows the detection of anti-HAV antibodies created by vaccine induced immune response.

### Detailed description of the present invention

In a first aspect, the present invention relates to a method for differentiation between infection-based immune response against an infective agent and a vaccine induced immune response against an infective agent in an individual whereby the vaccine is a vaccine based on the infective agent containing an antigen having conformational epitope(s), comprising the steps of:
- providing multiple of antigens derived from the infective agent wherein at least one antigen is provided having a linear epitope and at least one antigen is provided having a conformational epitope whereby the conformational epitope is present in the vaccine;
- allowing formation of binding pairs of an antibody present in a sample obtained from the individual with one of the multiple of antigens provided;
- determining binding pairs of antigen/antibody formed in said sample by an immunological method;
- differentiating infection based immune response and vaccine based immune response against the infective agent i) based on the presence and/or amount of binding pairs of antigen/antibody whereby the antigen has a linear epitope, and ii) based on the presence and/or amount of binding pairs of antigen/antibody whereby the antigen has the conformational epitope;
wherein the determination of the binding pairs is with the same type of immunological method, preferably within a single assay.

That is, the present inventors recognized that it is possible to differentiate between natural infection, namely, infection based immune response against an infective agent, and vaccine induced or based immune response against an infective agent based on the presence and/or the amount of antibodies recognizing antigen having a linear epitope of the infective agent.

While individuals having an immune response against the infective agent have antibodies identifying the antigen with the conformational epitope, the type and number of antibodies binding to antigens containing a linear epitope is reduced or for some antigens even absent in the vaccine based immune response in said individual.

Hence, an effective tool is provided for differentiating between natural infection and vaccine induced immune response in an individual.

As used herein, the term "virus" refers to whole virus also identified as virus particles and viral vector as well as virus like particles (VLPs). That is, vaccine composition may be composed of virus like particles. Virus like particles are particles resembling viruses but are not infectious because they do not contain any viral genetic material.

Further, the terms "comprise" or "comprising" as well as "contain" or "containing" which are used herein interchangeably include the embodiment of "consist" and "consisting of".

The term "antibody based method" refers to a method including detection of antibodies. That is, an antibody based method may include the embodiment of determining the presence of the antibody by means for binding to antibodies or to antigens bound to said antibodies.

Furthermore, the term "immunological method" refers to a method including the formation of a binding pair composed of antigen/antibody also known as antigen/antibody complex.

The present invention is based on the finding that determining antibodies in a sample from an individual directed against linear and conformational epitopes allow to distinguish infected and vaccinated immune response in said individual.

Not to be bound by theory, it is submitted that the vaccine based on inactivated or attenuated virus as well as parts of an infective agent being a microorganism is not processed inside the cell upon vaccination which results in a different antibody pattern compared to natural infection. In case of infection, the processing of viral antigens upon the intracellular replication of virus results in antibodies against linear epitopes. Regarding attenuated vaccines, it is submitted that due to the low rate of propagation of said virus, the antibodies directed against linear epitopes are reduced or absent.

In an embodiment of the present invention, the method is a method wherein the vaccine containing said antigen having conformational epitope(s) is an inactivated or attenuated microorganism, in particular, an inactivated or attenuated whole virus. The microorganism may also include bacteria or other infective agents such as protozoa. Further parasites in general are within the scope of vaccine prevent-bale infective agents.

Of course, the vaccine containing the antigen having conformational epitope(s) may also be a vaccine containing full length antigens derived from the infective agents including diphtheria-toxin, tetanus-toxin, *pneumococcus* polysaccharides conjugated to carrier proteins, HPV L1 protein or HBV S-antigen.

It is submitted that upon vaccination with these infective agent derived components, including the above mentioned toxins etc., only antibodies against conformational epitopes are produced since the antigens are not processed within infected cells after vaccination.

That is, the virus may be selected from influenza virus, yellow fever virus, human papillomavirus, hepatitis virus, polio virus, rotavirus, rubella virus, measles virus, mumps virus, varicella zoster virus, cytomegalovirus, Ebstein-Barr- virus, rabies virus. Hepatitis virus includes hepatitis A virus, hepatitis B virus, hepatitis C virus, hepatitis D virus, hepatitis E virus.
In another embodiment of the present invention, the vaccine is a vaccine based on the infective agent containing said antigen having conformational epitopes including toxoid, polysaccharide present on carrier proteins, as well as surface antigens, in particular diphtheria toxin, tetanus-toxin, *pneumococcus* polysaccharides conjugates carrier protein, HPVL1 protein or HBV S-antigen.

In an embodiment of the present invention, the immunological method for determining the binding pairs is conducted in a single assay, e.g. in a multiplex analysis. Suitable example for multiplex analysis or multiplex serology which are used herein interchangeably unless indicated otherwise, include the Luminex technology, e.g. the multiplexing technology platform xMAP. The skilled person is well aware of suitable Luminex assays including the xMAP technology as well as the MagPlex technology of Luminex. These technologies are based on microspheres. As used herein, the term "microspheres" includes the embodiments of beads. Generally, microspheres are small spherical particles with diameters in the micrometre range, typically in the range of 1 µm to 1000 µm. The microspheres also known as micro-particles include microbeads. The immunological method applied in the method according to the present invention may be a multiplex analysis or an ELISA based analysis. In case of multiplex analysis, the multiplex analysis may be based on microspheres, like the system MagPlex and/or the xMAP technology of Luminex.

In an embodiment of the present invention, the infective agent is a virus, like the virus mentioned above including hepatitis virus, measles virus, rubella virus and mumps virus.

In another embodiment, the at least one antigen having a linear epitope used in the method according to the present invention is a recombinant antigen derived from the infective agent. That is, the antigen is produced recombinantly by known techniques.

Further, an embodiment of the present invention relates to a method wherein the immunological method for determining the binding pairs of antigen antibody, the antigen antibody complex, is a multiplex analysis based method while the infective agent is a virus and the vaccine containing the confirmation epitope(s) is an inactivated virus or attenuated virus as active principle.

The multiple of antigens provided in the method according to present invention comprise at least one antigen having a linear epitope and at least one antigen having a conformational epitope whereby the conformational epitope is present in the vaccine. For example, the number of antigens having a linear epitope maybe 2, 3, 4, 5, 6 or even more. In addition, the number of conformational epitopes may be at least 1, like 2, 3, 4, 5 or 6.

In an embodiment, the number of antigen having a linear epitope are at least 4, like at least 5, as at least 6 while the number of antigen having a conformational epitope is 1.

The skilled person is well aware of suitable buffers and system for providing the multiple of antigens and for performing the steps identified. In particular, the skilled person is well aware of suitable systems including buffer for allowing information of the antigen/antibody complex, namely, the binding pairs of the antibody and antigen as identified.

Regarding the determination of the binding pairs, the determination can be effected by known devices typically, based on systems using optical measures like the measurement of mean fluorescence intensities. In addition, the system, e.g. a FACS, may allow determination of the scattered light, thus, allow determination of the size while the laser present in the FACS system allow to determine the formation of the antibody/antigen complex. Alternatively, e.g. using the Luminex system, a dual laser system is used wherein coloured coded beads are present. For each antigen provided, a type of bead is used being specifically colour encoded.

With the immunological method applied, the presence or absence of the binding pair may be determined. In addition, not only the presence or absence but also the level or amount of the formed binding pairs may be determined. Depending on the cutoff value or the threshold value applied, a positive or negative result may be obtained. That is, typically validation of the method according to the present invention may be conducted in so far that references are used with known status, namely, known status regarding natural infection or vaccine based immune response. A cut point is determined, namely, a cutoff or cut point (also known as threshold) allowing to differentiate between a positive result for the natural infection and a negative result for the vaccine based immune response. That is, based on the mean fluorescence intensity, a cut point is set for samples being positive for the antigen and samples being regarded as negative for the antigen, accordingly. This is conducted particularly in case of the antigen having a linear epitope.

In an embodiment of the method according to the present invention, the number of antigens having a linear epitope is at least two and wherein when no or less than 50% of the antigens having a linear epitope are determined positive for forming a binding pair with antibodies present in the sample of the individual while a binding pair is formed of the antigen having a conformational epitope with an antibody present in the sample of the individual, said individual has a vaccine based immune response against the infective agent.

In contrast, when at least 50% of the antigens having a linear epitope are identified positive for forming a binding pair with antibodies present in the sample of the individual while a binding pair formed of the antigen having a conformational epitope with an antibody present in the sample of an individual, said individual has an infection based immune response against the infective agent.

In an embodiment, the antigens are provided in a form of an array of said antigens. For example, the antigens may be provided as a microarray of antigens. The skilled person is well aware of suitable kind of arrays. For example, the array may be in form of a protein or peptide microarray. Alternatively, the array may be a mixture of beads having different colour, size and/or density, thus, allowing differentiation with suitable means including FACS and other measuring devices.

In another embodiment, the present invention comprises the step of binding of the different antigens to different types of microspheres including beads. These microspheres may be different between each other based on colour, size or density.

Determination of bound antibody may be conducted by usual measures typically, a secondary antibody is used, namely, an anti-human antibody in case of human individuals. The secondary antibody may contain marker moieties. That is, commercially available detection systems including these types of antibodies may be used for determining the antigen antibody complexes, namely, the binding pairs mentioned.

With the method according to the present invention simultaneous analysis of antibodies against multiple antigens is possible, thus, allowing measurement of antibody pattern. In addition, measurement of large numbers of serum samples is possible.

In another aspect of the present invention, a kit or system for differentiating between infection based immune response against an infective agent and a vaccine induced immune response against an infective agent in an individual, comprising multiple of antigens derived from the infective agent wherein at least one antigen is provided having a linear epitope and at least one antigen is provided having a conformational epitope whereby the conformational epitope is present in the vaccine; means for determining antibodies present in a sample of an individual whereby the antibodies are determined as binding pairs of antigens and antibodies and whereby said means have optionally a marker moiety for measuring the presence and/or amount of the formed binding pair of antigen/antibody; and, optionally, means for binding the antigens is provided.

That is, the kit or system according to the present invention for use in differentiating between infection based immune response and vaccine based immune response in the individual comprise the multiple of antigens. These multiple of antigens may be provided in form of an array, like a microarray. Alternatively, these multiple of antigens may be provided together with means for binding the antigens, e.g. microspheres like beads. Further, said kit or system comprises means for determining antibodies present in a sample of an individual whereby the antibodies are determined as binding pairs of antigens and antibodies. The means include secondary antibodies directed against human antibodies in case the individual is a human. Suitable means are well known to the skilled person. Optionally, these means may contain additionally a marker moiety for measuring the presence and/or amount of the formed binding pair of antigen/antibody. Suitable marker moieties include known label like fluorescence label, radioactive label but also biotin, avidin or streptavidin and the like.

The kit or system according to the present invention is useful for conducting a method according to the present invention.

The kit or system is especially adapted for multiplex analysis, in particular, Luminex based technology. That is, the kit or system include microspheres or beads as used in the multiplex analysis, for example, differently coloured beads or differently sized beads.

Finally, the present invention relates to an array, like a microarray, for multiplex analysis, containing at least two antigens derived from an infective agent wherein at least one antigen is an antigen having a linear epitope and wherein at least one antigen is an antigen having a conformational epitope of said infective agent, and said array, like said microarray, is a microsphere based array, like a microsphere based microarray.

In case of using a bead mix suitable for the multiplex analysis as described by Luminex, it is possible to determine in one assay antibodies against a multiple of antigens, for example up to 500 antigens, simultaneously. Thereby small amounts of serum is required keeping the costs low.

The present invention provides a valuable tool for efficient population based serosurveys and also for individual differential diagnosis. In particular, the present invention provides a one assay system for differentiation while the prior art requires more than one assay and, even more than one immunological method.

The following examples illustrate the invention further without limiting the same to a specific embodiment described in the examples.

### Example 1

### Serum

For the validation of the assay we used defined serum samples with known serological HAV status (HAV infected, HAV negative). These samples were provided and IgG status confirmed by the national reference centre for HAV in Regensburg, Germany. Serum samples from vaccinated individuals, collected in a study performed at the HZI (ethical approval, # 2198-2014 MHH Hannover, Germany), were verified with vaccination card and standard reference ELISA for IgG positivity (Hepatitis A virus Ab ELISA Kit (KA0284, Abnova). HAV status determination done by the reference center and with the Abnova ELISA, respectively are considered as gold standard.

Validation set 1 (antigen cutoff) consisted of 50 anti HAV IgG negative sera and 10 HAV RNA positive sera. Validation set 2 (assay cutoff) consisted of 57 IgG negative samples, 34 IgG positive serum samples of infected individuals and 42 IgG positive serum samples of vaccinated individuals.

### Antigens.

The nucleotide sequence NC_001489.1 (NCBI Reference Sequence) for HAV strain HM175 was used. Full length coding sequences of structural HAV proteins VP1 (bp 2208 - 3107), VP2 (bp 804 - 1469) and VP3 (bp 1470-2207) and non-structural HAV proteins 2A (bp 3108 - 3674), 2C (3996 - 5000) and 3C (bp 5292 - 5948) were commercially synthesized (MWG) and expressed as double fusion proteins with N-terminal GST and a C-terminal peptide (tag) consisting of the 11 C-terminal amino acids from the large T antigen of SV40. The mature peptide without the GST and tag are shown in Seq. ID No. 1 (VP1), Seq. ID No. 2 (VP2), Seq. ID No. 3 (VP3), Seq. ID No. 4 (2A), Seq. ID No. 5 (2C) and Seq. ID No. 6 (3C).The pGex-Tag backbone vector and cloning has been described by Sehr, P. et al. (2001) J. Immunol. Methods 253, 153-162. All clones were verified by sequence analysis. Fusion proteins were expressed in *E. coli* BL21. Successful full-length antigen expression was verified by Western Blot and anti-tag ELISA as described by Sehr, P. et al. (2001) J. Immunol. Methods 253, 153-162.

### Glutahione-Casein (GC)

GC was produced as described previously (Sehr, P. et al. (2001) J. Immunol. Methods 253, 153-162). Casein (Sigma) at a concentration of 5 mg/ml in phosphate-buffered saline (PBS) was incubated for 15 min at RT with 0.4 mM *N-*ethylmaleimide (NEM, Sigma) to block the single cysteine residue in casein Thereafter 4 mM sulfosuccinimidyl 4-[p-maleimidophenyl]butyrate (SSMBP, Pierce, Rockford, IL) was added as crosslinker and the reaction proceeded for 30 min at room temperature. Free SSMBP and NEM were separated from casein by size exclusion chromatography on PD10 columns (GE Healthcare, 17-0851-01) The protein fraction was then supplemented with 10 mM glutathione (Sigma) and the coupling reaction was executed for 1 h at RT. The glutathione casein was separated from unbound glutathione by gel filtration with PD10, using PBS as buffer and stored at -20°C in small aliquots.

### Covalent Coupling of GC and HAV whole virus to Luminex Microspheres

GC was then coupled to spectrally distinct fluorescence labeled magnetic beads (MagPlex; Luminex) using N-Hydroxysuccinimide and 1-(3-Dimethylaminopropyl)-3-Ethylcarbodiimide (NHS/EDC) for activation following description by Waterboer, T. et al. (2005) Clin. Chem. 51, 1845-1853. Deviating from the described protocol we used a magnetic separator (Dyna Mag™-2, Life technologies) instead of centrifugation for the washing steps, alternating pelleting of the beads, aspiration of the supernatant and adding 1 ml activation buffer.
Covalent coupling of GC to microspheres allows interchangeable use of microspheres and GST tagged recombinant antigens in bead mix.
Whole formalin inactivated HAV virus (Aviva Systems Biology, Cat # OPMA04543) was coupled to magnetic beads using AMG™ Activation Kit for Multiplex Microspheres according to the manufacturer's protocol. Magnetic beads were activated for 1 hour at room temperature with Mix&Go™ activation reagent. Activated magnetic beads were incubated with 200 µg formalin inactivated HAV virus for 1 hour at room temperature. HAV coupled magnetic beads can be stored at 4°C in storage buffer.

*Multiplex serology.* Multiplex serology (MUSE) was performed as described previously (Waterboer, T. et al. (2005) Clin. Chem. 51, 1845-1853). The complete bead set consisted of beads presenting recombinant antigens and beads coupled with whole inactivated HAV virus. VP1, VP2, VP3, 2A, 2C and 3C - GST fusion proteins were loaded and affinity-purified on glutathione-casein coupled beads. Serum samples were incubated at 1:100 final dilution with pooled bead set in 96 well flat bottom plates (Greiner, 655101). All plate wash steps were performed with Tecan hydroflex 96-Well washer (Tecan Austria GmbH). Bound antibodies were quantified with biotinylated goat anti-human IgA, IgM, IgG (Dianova), and R-phycoerythrin-labeled streptavidin (Roche) in a Luminex 100 analyzer as the median R-phycoerythrin fluorescence intensity (MFI) from at least 100 beads of the same bead set.

### HAV ELISA

We used Hepatitis A virus Ab ELISA Kit (KA0284, Abnova) for confirmation of antibody status in vaccinated serum samples according to the manufacturer's protocol (Gold standard).

*Validation.* Antigen-specific MFI values were calculated using a set of standard sera with validated status (Shankar, G., et al. (2008) J. Pharm. Biomed. Anal. 48, 1267-1281). In short, 50 IgG negative serum samples were measured five times in total, by two analysts. After transformation into log scale and exclusion of outliers (+/- 1.5 IQR) normal distribution was analyzed for all antigens. Mean and variance were estimated using ANOVA. With a same mean a fixed cutoff is calculated for antigens, else a floating cutoff (normalization factor + run specific mean for normalization sera) is used. The antigen specific cutoff is the back transformed mean + 1.645 Standard deviation (SD). A sample with a MFI > cutoff is positive for the antigen, a samples with a MFI ≤ cutoff negative for the antigen. Validation set 2 was used to calculate the assay cutoff. All statistical analysis was done using SAS 9.2.

HAV Multiplex serology was performed on 50 confirmed anti-HAV-IgG negative sera and the cutoff (mean + 1.645 SD) and normalization factor, respectively are depicted for each antigen in table 1.

| **ANTIGEN** | **Normal-distribution** | **Same mean** | **Same variance** | **Normalization factor** | **Fixed Cutoff** |
|---|---|---|---|---|---|
| VP1 | YES | NO | YES | 1.23 | (274) |
| VP2 | YES | NO | YES | 0.66 | (372) |
| VP3 | YES | NO | YES | 0.92 | (347) |
| 2A | YES | NO | YES | 1.80 | (803) |
| 2C | YES | NO | NO | 1.20 | (215) |
| 3C | YES | YEs | YES | - | 164 |
| HAV | YES | Yes | YES | - | 1138 |

Results of the statistical evaluation show that a floating cutoff is needed for 5 of the 6 recombinant antigens. The floating cutoff is calculation in each run via the mean of up to ten normalization sera with negative HAV status plus the normalization factor.

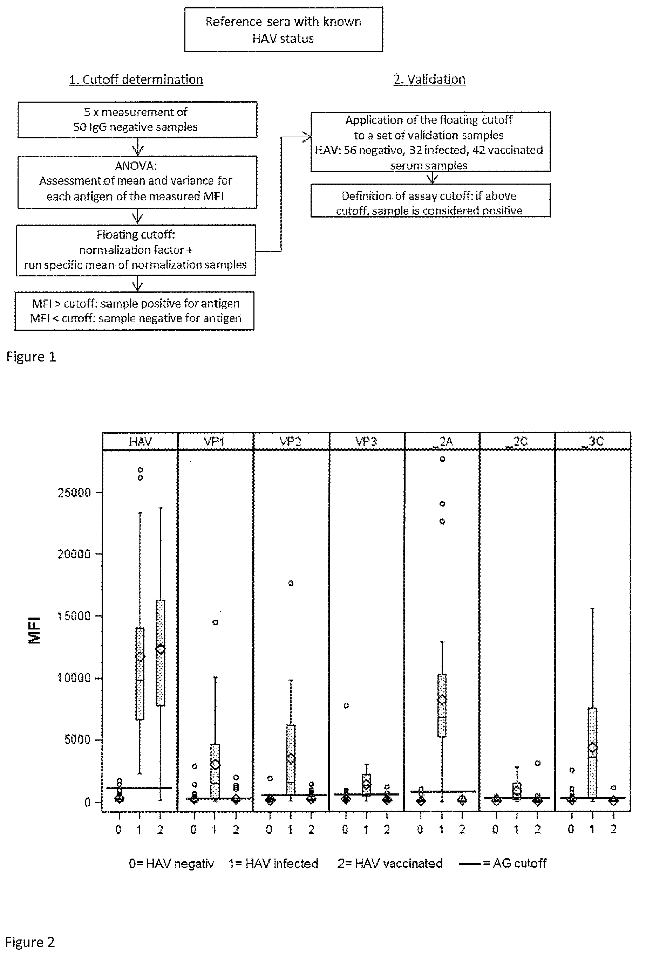

A signal above the cutoff is considered antibody positive for the respective antigen. Applying HAV multiplex serology on validated infected and vaccinated sera shows that infected sera contain antibodies reactive to in *E.coli* expressed recombinant viral antigens VP1 - 3C, while vaccinated sera do not react significantly (Figure 1).

This result was unexpected as literature results suggested vaccinated and infected sera can be distinguished by showing i) binding of antibodies from both vaccinated and infected serum samples against recombinant structural recombinant proteins (VP1, VP2, VP3) but only showing ii) binding of antibodies against the non-structural HAV-protease 3C for infected serum.

HAV antigens are produced by mammalian cells upon infection. Their expression in E.coli cells can result in incorrect folding. Furthermore, whole virus presents conformational epitopes consisting of amino acid stretches overlapping VP1, VP2 and VP3, respectively, thus generating epitopes not present on the individual antigen.

We solved the lacking presentation of conformational epitopes by coupling whole formalin inactivated HAV to magnetic beads. Antibodies from infected serum samples bind with different specificity and sensitivity to the individual recombinant HAV antigens, as seen in table 2.

Table 2: Sensitivity and Specificity for individual recombinant HAV antigens allowing differentiation for HAV negative and infected serum samples, only.

| Floating cutoff | VP1 | VP2 | VP3 | 2A | 2C | 3C |
|---|---|---|---|---|---|---|
| Sensitivity (%) | 77 | 96 | 71 | 97 | 74 | 74 |
| Specificity (%) | 83 | 86 | 92 | 93 | 89 | 94 |

HAV antigen 2A by itself is able to distinguish HAV infected from negative sera with a sensitivity and specificity of 97% and 93 %, respectively.

For HAV multiplex serology, the assay cutoff which allows the best differentiation between infected, vaccinated and negative serostatus is composed as follows:
a) Antibody positivity against HAV whole virus and at least any three out of six recombinant antigens is defined as HAV infected serum sample.
b) Antibody positivity against HAV whole virus and maximum any two out of six recombinant antigens is defined as HAV vaccinated serum sample
c) Antibody negativity against HAV whole virus is defined as HAV negative serum sample

Applying the assay cutoff allows the determination of serostatus, as shown in table 3.

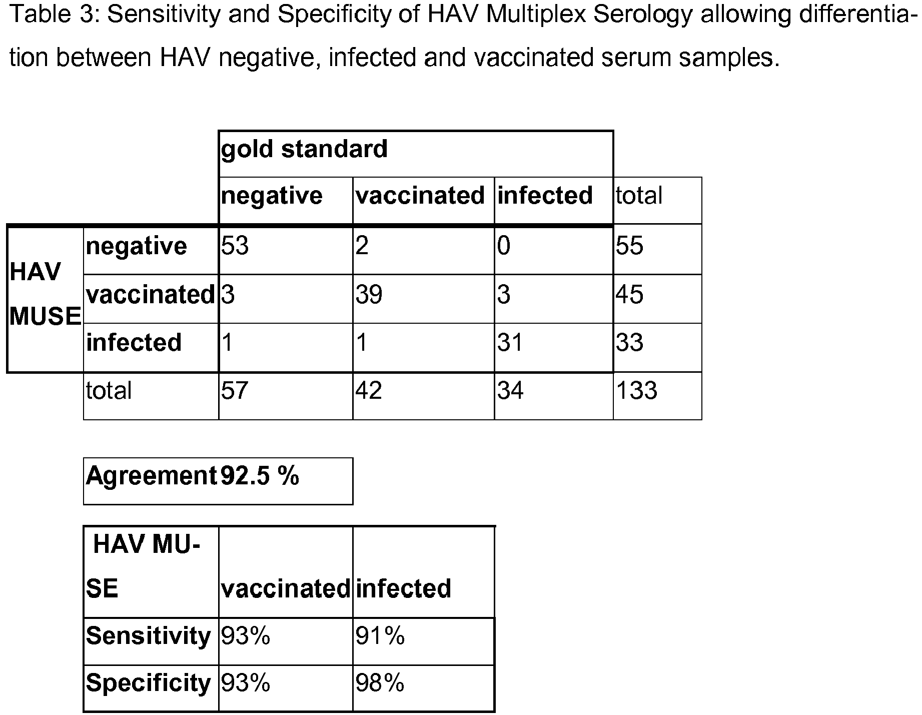

Result for Validation serum samples using an assay cutoff of 3/6. Any three of the six antigens VP1, VP2, VP3, 2A, 2C and 3C have to be positive for a serum to be considered 'antigen positive' and therefore 'HAV infected'. A serum positive for HAV whole virus but 'antigen negative' is considered 'HAV vaccinated'. Serum samples 'antigen negative' and HAV whole virus negative are considered HAV negative.

## Claims

1. An antibody based method for differentiation between infection-based immune response against an infective agent and a vaccine induced immune response against an infective agent in an individual whereby the vaccine is a vaccine based on the infective agent containing an antigen having conformational epitope(s), comprising the steps of:
- providing multiple of antigens derived from the infective agent wherein at least one antigen is provided having a linear epitope and at least one antigen is provided having a conformational epitope whereby the conformational epitope is present in the vaccine;
- allowing formation of binding pairs of an antibody present in a sample obtained from the individual with one of the multiple of antigens provided;
- determining binding pairs of antigen/antibody formed in said sample by an immunological method;
- differentiating infection based immune response and vaccine based immune response against the infective agent i) based on the presence and/or amount of binding pairs of antigen/antibody whereby the antigen has a linear epitope, and ii) based on the presence and/or amount of binding pairs of antigen/antibody whereby the antigen has the conformational epitope;
wherein the determination of the binding pairs is with the same type of immunological method, preferably within a single assay.

2. The method according to claim 1 wherein the vaccine containing said antigen having conformational epitope(s) is an inactivated or attenuated microorganism, in particular, an inactivated or attenuated whole virus.

3. The method according to any one of the preceding claims wherein the immunological method is an immunological method selected from multiplex analysis and ELISA.

4. The method according to any one of the preceding claims wherein the at least one antigen having a linear epitope is at least one recombinant antigen derived from the infective agent.

5. The method according to any one of the preceding claims wherein the infective agent is a virus.

6. The method according to claim 5 wherein the infective agent is a virus selected from hepatitis virus including hepatitis A, hepatitis B, hepatitis C, hepatitis D, hepatitis E, measles virus; rubella virus; mumps virus, influenza virus, human papilloma virus, varicella zoster virus, poliovirus, yellow fever virus, rotavirus, cytomegalovirus, Ebstein-Barr- virus, rabies virus.

7. The method according to any one of the preceding claims wherein the immunological method is a multiplex analysis based method, the infective agent is a virus and the vaccine containing the conformational epitope(s) contains inactivated or attenuated virus as active principle.

8. The method according to any one of the preceding claims wherein the number of antigens having a linear epitope is at least two and wherein when no or less than 50% of the antigens having a linear epitope are determined positive for forming a binding pair with antibodies present in the sample of the individual while a binding pair formed of the antigen having a conformational epitope and an antibody present in the sample of the individual is determined, said individual has a vaccine based immune response against the infective agent.

9. The method according to any one of the preceding claims wherein when at least 50% of the antigen having a linear epitope are identified positive for forming a binding pair with antibodies present in the sample of the individual while a binding pair formed of the antigen having a conformational epitope and an antibody present in the sample of the individual is determined, said individual has an infection-based immune response against the infective agent.

10. The method according to any one of the preceding claims wherein the antigens are provided in form of an array, like a microarray, of said antigens.

11. A Kit or a system for differentiating between infection based immune response against an infective agent and a vaccine induced immune response against an infective agent in an individual, comprising multiple of antigens derived from the infective agent wherein at least one antigen is provided having a linear epitope and at least one antigen is provided having a conformational epitope whereby the conformational epitope is present in the vaccine; means for determining antibodies present in a sample of an individual whereby the antibodies are determined as binding pairs of antigens and antibodies and whereby said means have optionally a marker moiety for measuring the presence and/or amount of the formed binding pair of antigen/antibody; and, optionally, means for binding the antigens.

12. The kit or system according to claim 11 for conducting a method according to any one of claims 1 to 10.

13. The kit or system according to claim 11 or 12 wherein the different antigens are present in form of an array, like a microarray, bound to said means in form of microspheres, in particular, coloured microspheres, wherein each antigen is bound to a predetermined type of coloured microspheres.

14. The kit or system according to any one of claims 11 to 13 wherein the kit or system is suitable for multiplex analysis, in particular, Luminex based technology.

15. An array, like a microarray, for multiplex analysis, containing at least two antigens derived from an infective agent wherein at least one antigen is an antigen having a linear epitope and wherein at least one antigen is an antigen having a conformational epitope of said infective agent, and said array, like said microarray, is a microsphere based array, like a microsphere based microarray.
